**Europäisches Patentamt**

**European Patent Office**

**Office européen des brevets**

(19)

(11) Veröffentlichungsnummer: **0 195 094**

**A1**

(12) **EUROPÄISCHE PATENTANMELDUNG**

(21) Anmeldenummer: 85103044.5

(22) Anmeldetag: 16.03.85

(51) Int. Cl.⁴: **C 12 M 1/12**
C 12 P 7/06, C 12 P 7/16
C 12 P 7/28

(43) Veröffentlichungstag der Anmeldung:
24.09.86  Patentblatt  86/39

(84) Benannte Vertragsstaaten:
AT DE FR GB IT NL SE

(71) Anmelder: **Starcosa GmbH**
**Am Alten Bahnhof 5**
**D-3300 Braunschweig(DE)**

(72) Erfinder: **Tegtmeier, Uwe, Dr.**
**Eichenweg 5**
**D-3341 Wittmar(DE)**

(74) Vertreter: **Döring, Rudolf, Dr.-Ing.**
**Patentanwälte Dr.-Ing. R. Döring Dipl.-Phys. Dr. J. Fricke**
**Jasperallee 1a**
**D-3300 Braunschweig(DE)**

(54) Verfahren zur fermentativen Erzeugnung oganischer Lösungsmittel wie Butanol, Azeton, insbesondere von Ethanol.

(57) Das Verfahren zur fermentativen Erzeugung organischer Lösungsmittel, insbesondere von Ethanol, durch kontinuierliche Fermentation eines zucker- oder stärkehaltigen wässrigen Medium, bei dem Ethanol sowohl als Stoffwechselprodukt von Hefen als auch von Bakterien gebildet wird, sieht eine Trennung des Ethanols aus dem Prozeß mittels zweier Stoffablaufströme unterschiedlicher Zusammensetzung vor. Der eine Stoffstrom (7) stellt das Permeat dar, welches durch Membranfiltration (2) gewonnen wird und weitestgehend frei ist von Hefe- und/oder Bakterienzellen sowie Feststoffpartikeln, während der andere Stoffstrom (8) als Fermenter-Stoffstrom direkt aus dem Fermentationsfluid entnommen wird und den größten Teil der nichtlöslichen Substanzen sowie die produktbildende Biomasse enthält und gegenüber dem Permeat-Stoffstrom (7) kleiner gehalten wird.

Es wird eine hohe aktive Zellkonzentration des produzierenden Mikroorganismusses aufrechterhalten und eine hohe Produktivität bei unsteriler Prozeßführung erreicht.

Croydon Printing Company Ltd.

EP 0 195 094 A1

Starcosa GmbH,
Am Alten Bahnhof 5
3300 Braunschweig

Verfahren zur fermentativen Erzeugung organischer
Lösungsmittel wie Butanol, Azeton, insbesondere
von Ethanol.

Die Erfindung betrifft ein Verfahren zur fermentativen
Erzeugung organischer Lösungsmittel wie Butanol, Azeton,
insbesondere von Ethanol, durch kontinuierliche Fermentation eines zucker- oder stärkehaltigen wässrigen
Mediums, welches neben den fermentierbaren auch lösliche
und nichtlösliche nichtfermentierbare Substanzen enthalten kann, wobei die organischen Lösungsmittel, insbesondere Ethanol, sowohl als Stoffwechselprodukte von
Hefen als auch von Bakterien gebildet werden.

Durch die Verknappung der Weltreserven an fossilen
Energieträgern sind Alternativ-Energieträger interessant
geworden, die vollständig aus nachwachsenden pflanzlichen Rohstoffen hergestellt werden können. In diesem
Zusammenhang hat insbesondere Ethanol als Industriegrundstoff ebenso wie als Treibstoff seit einiger Zeit
zunehmend an Bedeutung gewonnen.

Neuere Verfahren energetisch aufwendiger Teilprozesse, wie Rohstoff-Aufbereitung, Produktabtrennung und -konzentrierung führten dazu, daß der Anteil an aufzuwendender Energie im Gesamtproduktionsprozeß deutlich kleiner wurde als der Energieinhalt des gewonnenen Ethanols, so daß die Herstellung vom energetischen Standpunkt aus sinnvoll ist.

Um das Verfahren zur Gewinnung von Ethanol wirtschaftlich zu gestalten ist es notwendig, eine möglichst hohe Substratausbeute zu erzielen.

In batch-Fermentationen betragen die Ausbeuten infolge des Substratverbrauches für die Nachbildung von Zellmasse lediglich ca. 92 % des theoretischen Wertes.

Bei Verfahren der einleitend genannten Art zur kontinuierlichen Produktion von Ethanol auf fermentativem Wege müssen bisher erhebliche Nachteile in Kauf genommen werden.

So ist eine mechanische Abtrennung der Biomasse aus dem ablaufenden Stoffstrom lediglich bei reinen feststofffreien Substraten möglich. Ebenso können flockulierende Hefen oder immobilisierte Zellen, die sedimentierende Eigenschaften aufweisen, nur rohstoffabhängig eingesetzt werden. Wenn aber Biomasse nicht abgetrennt werden kann, ergeben sich hohe Ausbeuteverluste, da hierdurch bedingt größere Mengen an Biomasse erzeugt werden müssen.

Auch bei reinen substrathaltigen Medien lassen sich mit konventionellen Hefeabtrenn-Methoden und Rückführung der Zellmasse in das Fermentationsfluid bzw. den Fermenter nur aktuelle Zellkonzentrationen von etwa 50 g Hefetrockensubstanz pro Liter einstellen. Entsprechend gering sind die Produktivitäten, d.h. die Raum/Zeitausbeuten an Produkt und/oder die Produktkonzentration.

Es ist ferner eine energieaufwendige Sterilisation des Substrates erforderlich, da eine infektionsarme Betriebsweise nur hierdurch gewährleistet werden kann. Z.Zt. ist kein kontinuierliches Fermentationsverfahren der einleitend genannten Art bekannt, das ohne in-line-Sterilisation über längere Zeit arbeitet.

Der Erfindung liegt die Aufgabe zugrunde, ein Verfahren der einleitend genannten Art so auszubilden, daß eine kontinuierliche Fermentation zur Erzeugung des genannten Lösungsmittels, vorzugsweise von Ethanol, ermöglicht, bei der durch Aufrechterhalten einer hohen aktiven Zellkonzentration des produzierenden Mikroorganismusses eine mit diskontinuierlicher Fermentation vergleichbare Produktkonzentration, eine hohe Produktivität und eine unsterile Prozeßführung erreicht werden.

Gelöst wird die vorstehende Aufgabe erfindungsgemäß durch die im kennzeichnenden Teil des Hauptanspruches aufgeführten Merkmale.

Durch das neue Verfahren ergeben sich relativ geringe Verluste an Biomasse, da bei der Abtrennung des Permeatstoffstromes durch die Filtration die Biomasse im Fermentationsfluid erhalten bleibt und in den Fermentationsprozeß zurückgeführt wird und nur ein kleiner Anteil der Biomasse durch den bezogen auf den Permeat-Stoffstrom kleiner gehaltenen Fermenter-Stoffstrom abgezogen wird. Dies ist zur Erhaltung eines hohen Aktivitätsgrades der produktbildenden Biomasse erforderlich, jedoch stellt diese abgezogene Biomasse den einzigen prozeßbedingten Verlust dar, so daß Produktionsausbeuten mit dem neuen Verfahren in der Größenordnung von 95 % des theoretischen Wertes erreicht werden. Dabei wird durch die Größe des kontinuierlich abgezogenen Fermenter-Stoffstromes die Gesamttrockensubstanz-Konzentration im Fermentationsfluß begrenzt.

Besonders günstige Ergebnisse erzielt man, wenn der Permeat-Stoffstrom ein Vielfaches des Fermenter-Stoffstromes beträgt, da hierdurch der Anteil der kontinuierlich abgeführten Biomasse besonders gering ist und damit eine Steigerung der Produktausbeute einhergeht.

Weitere Einzelheiten und zweckmäßige Weiterbildungen der Erfindung ergeben sich aus den Unteransprüchen 3 bis 7.

Die Gewinnung des Permeat-Stoffstromes wird vorzugsweise mit einer Mikrofiltrations-Hollow-Fibermembran vorgenommen. Durch die Filtrationseinheit werden die wässrigen, mit einem gewissen Feststoffpartikel- und Schmutzanteil belasteten Fermentationsfluide durchge-setzt. Membranen, die als Hollow-Fiber-Membranen ausgebildet sind, können ohne aufwendige Vorfiltration mit einem solchen Fermentationsfluid beschickt werden, da durch die sich ausbildende turbulente Rohrströmung ein Fouling der Membranen weitestgehend unterdrückt werden kann.

Um die Druckverluste der Hollow-Fiber-Membranen bei gleichzeitig hoher spezifischer Permeatrate möglichst gering zu halten, ist bei der Auswahl auf günstige geometrische Daten zu achten. So sollte in Abhängigkeit von der Viskosität des Fermentationsfluides das Verhältnis Fiber-Innendurchmesser zu Fiber-Länge so gewählt werden, daß der mittlere Strömungsdruckverlust der notwendigen transmembranen Druckdifferenz entspricht. Hierdurch werden die Pumpenleistungen erheblich reduziert.

In dem Fermenter-Stoffstrom sind nahezu alle Bestandteile der durch das zucker- oder stärkehaltige wässrige
Medium zugeführten nichtlöslichen, nichtfermentierbaren
Substanzen und die Biomasse enthalten, die aufgrund der
Ausschlußgrenze der verwendeten Membran nicht im
Permeat-Stoffstrom enthalten sind. Hierzu gehören alle
nichtgelösten Bestandteile, flockuliertes Eiweiß,
autolysierte Zellen und aktive Biomasse. Von letzterer
geht gemäß den obigen Ausführungen nur ein geringer Teil
durch den kontinuierlich abgezogenen Fermenter-Stoffstrom
verloren.

Die erfindungsgemäß vorgesehene Aufteilung der aus dem
Fermentationsprozeß abgezogenen getrennten unterschiedlichen Stoffströme führt zu dem weiteren Vorteil einer
erheblichen Erleichterung und Verbesserung der Entsorgung
des Gesamtproduktionsprozesses. Es kann nämlich nach
Abtrennung des Lösungsmittels aus dem Permeat-Stoffstrom
dieser dem Abwasser zur Verwertung in einer Biogasanlage
zugeführt werden. Da sich die Beschickung von Biogasanlagen mit feststoffhaltigen Abwässern negativ auf ihre
hydraulische Belastbarkeit auswirkt, führt die Verarbeitung des weitestgehend feststoffreien und von dem gewonnenen organischen Lösungsmittel bzw. Ethanol befreiten Permeat-Stoffstromes zur einer entscheidenden Erhöhung der Produktivität der Biogasanlage.

Der sehr viel kleinere Fermenter-Stoffstrom kann nach
Entzug des erzeugten organischen Lösungsmittels eingedampft und abschließend getrocknet werden. Er stellt in
Abhängigkeit von dem verwendeten Rohstoff ein hochwertiges Futtermittel dar.

Ein weiterer Vorteil des erfindungsgemäß ausgebildeten Verfahrens liegt darin, daß hohe Gesamttrockensubstanzgehalte des Fermentationsfluids auch mit sehr niedrig konzentrierten zucker- oder stärkehaltigen Substanzen erzielt werden.

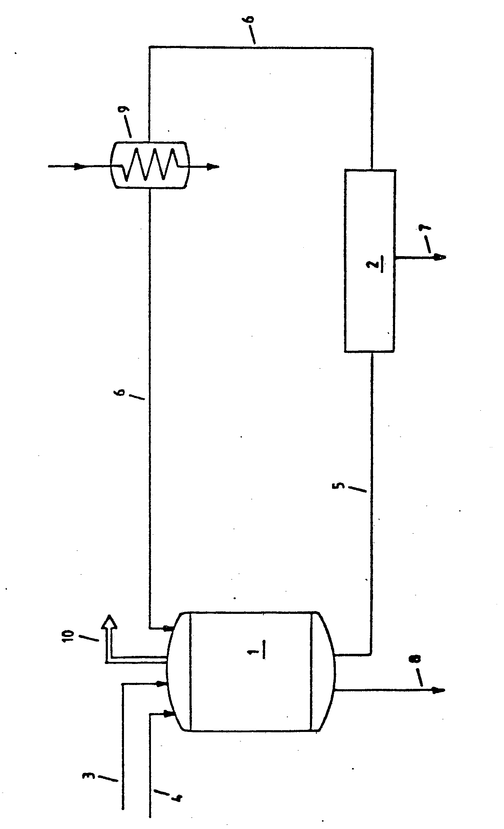

Die beigefügte Zeichnung gibt ein prinzipielles Fließ-schema für das Verfahren nach der Erfindung wieder.

Der kontinuierlich arbeitende Fermenter 1 wird über die Zuleitung 3 kontinuierlich mit unsteriler zucker- oder stärkehaltiger Substanz und über die Zuleitung 4 mit einer bestimmen Wassermenge beschickt, die abhängig ist von der Konzentration der zucker- oder stärkehaltigen Substanz. Über eine Verbindungsleitung 5, welche von dem Fermentationsfluid durchströmt wird, ist der Fermenter 1 mit einer Membraneinheit 2 verbunden. In der Membraneinheit 2 erfolgt eine Trennung des Fermentationsfluids in einen weitestgehend feststofffreien Permeat-strom, welcher über die Leitung 7 aus der Membraneinheit 2 abgezogen wird. Das aus der Membraneinheit 2 abfließende Fermentationsfluid wird über die Leitung 6 durch einen Wärmetauscher 9 hindurchgeleitet und in den Fermenter 1 zurückgeführt. Da der aus der Membraneinheit 2 abgezogene Permeat-Stoffstrom im Verhältnis zu dem die Membraneinheit durchströmenden Fermentationsfluid gering ist, ergibt sich für das aus der Membraneinheit 2 durch die Leitung 6 abfließende Fermentationsfluid eine nur unwesentlich geringe Anreicherung an Feststoffpartikeln und Biomasse. In dem Wärmetauscher 9 wird die Fermentationswärme weitestgehend abgeführt. Dies ermöglicht eine einfachere Bauart des Fermenters 1.

Aus dem Fermenter 1 wird über die Leitung 8 der Fermenter-Stoffstrom abgezogen, der in seinen Konzentrationsverhältnissen dem Fermentationsfluid entspricht, wie es durch die Leitung 5 der Membranheit 2 zugeführt wird. Durch die biochemische Umsetzung der zucker- oder stärkehaltigen Substanz entsteht ein Reduktionsäquivalent, nämlich $CO_2$, welches in Gasform über die Leitung 10 den Fermenter verläßt.

Dem Verfahren nach der Erfindung liegt zugrunde, daß die aktive produktbildende Massenkonzentration Werte von mindestens 1oo g Trockensubstanz pro Liter erreicht.

Die Erfindung wird nachstehend an zwei Beispielen näher erläutert.

Beispiel 1

Für eine kontinuierliche Ethanol-Fermentation wird eine verdünnte Rübenmelasse als zuckerhaltige Substanz verwendet. Für die Gewinnung des Permeat-Stoffstromes wurde eine Hollow-Fiber-Cartridge der Enka AG, 5600 Wuppertal, Typ HB 3355 E21 eingesetzt. Das Material der Membranen ist Polypropylen, vergossen in Polyuretan.

Die nachstehende Tabelle gibt die Versuchsparameter und Ergebnisse wieder, wie sie als Dauerwerte erzielt wurden.

Tabelle 1

| | | | |
|---|---|---|---|
| Arbeitsvolumen Fermenter | | 65 | (1) |
| Durchsatzrate | ca. | 0,6 | (-) |
| Permeatrate (Hauptablaufstrom) | | 39,5 | (1/h) |
| Nebenablaufstrom | | 0,7 | (1/h) |
| EtOH-Konzentration | | 8,0 | (Vol. %) |
| Viskosität | | 4,8 | (mPs) |
| pH-Wert | ca. | 4,6 | (-) |
| Temperatur | | 38 | (°C) |
| HTS | | 110 | (g/l) |
| GTS | | 180 | (g/l) |
| aktuelle Zuckerkonzentration | ca. | 3 | (g/l) |

| substrathaltiges Medium | unsterile Melasse, 1:1 verdünnt mit Wasser | |
|---|---|---|
| Zuckerkonzentration (Saccharose) | 270 | (g/l) |
| Gesamttrockensubstanz | 350 | (g/l) |
| Volumenstrom | 18,5 | (l/h) |
| Mikroorganismus | Hefe Hett 80 | |
| Wachstumsrate | ca. 0,01 | (1/h) |
| spez. Gärrate | ca. 0,36 | (g EtOH/g HTS·h) |
| Aktivität | 97 | (%) |

Abkürzungen:

HTS = Hefetrockensubstanz

GTS = Gesamttrockensubstanz

Es zeigt sich, daß eine Produktivität von 49,2 Liter Ethanol pro m³ Fermentervolumen und Stunde bei hoher Produktkonzentration von 8 Vol.% in den Stoffströmen erreicht werden konnte. Der Druckverlust in der Hollow-Fiber-Cartridge betrug 0,35 bar, bei einer mittleren transmembranen Druckdifferenz von 0,4 bar. Aufgrund des geringen Zellmassenverlustes von ca. 80 g Hefetrockensubstanz pro Stunde konnten 3,22 Liter Ethanol pro Stunde aus 5 Kilogramm zugeführter Saccharose erzeugt werden. Dieser Wert entspricht 95 % der Theorie.

Beispiel 2

Eine kontinuierliche Ethanolfermentation wurde mit verdünnter Rübenmelasse als zuckerhaltige Substanz bei höheren Gesamttrockensubstanzgehalten betrieben. Es fand die gleiche Hollow-Fiber-Cartridge wie unter Beispiel 1 genannt Anwendung.

In nachstehender Tabelle 2 sind wiederum die signifikanten Parameter zusammengestellt.

Tabelle 2

| | | |
|---|---|---|
| Arbeitsvolumen Fermenter | 65 | (1) |
| Durchsatzrate | 0,6 | (-) |
| Permeatrate (Hauptablaufstrom) | 50,0 | (l/h) |
| Nebenablaufstrom | 0,55 | (l/h) |
| EtOH-Konzentration | 8,0 | (Vol.%) |
| Viskosität | 5,6 | (mPs) |
| pH-Wert | 4,6 | (-) |
| Temperatur | 38 | ($^{\circ}$C) |
| HTS | 135 | (g/l) |
| GTS | 230 | (g/l) |
| aktuelle Zuckerkonzentration | 2,5 | (g/l) |
| substrathaltiges Medium | unsterile Melasse, 1:1 verdünnt mit Wasser | |
| Volumenstrom | 23,2 | (l/h) |
| Mikroorganismus | Hefe Hett 80 | |
| Wachstumsrate | ca. 0,01 | (1/h) |
| spez. Gärrate | 0,38 | (g EtOH/g HTS·h) |
| Aktivität | 95 | (%) |

Abkürzungen:

HTS = Hefetrockensubstanz

GTS = Gesamttrockensubstanz

Aus den vorgenannten Werten ist zu erkennen, daß die Produktivität mit zunehmender aktueller Zellmassenkonzentration ansteigt. Es wurde bei 135 g Hefetrockensubstanz pro Liter ein Wert von 62,2 Liter Ethanol pro m$^3$ Fermentervolumen und Stunde erreicht. Durch Verringerung des Fermenter-Stoffstromes und Erhöhung des Eintrages der verdünnten Rübenmelasse konnte ebenfalls die Gesamttrockensubstanz auf 230 g pro Liter angereichert werden. Die übrigen Fermentations-Parameter sind vergleichbar mit Beispiel 1.

Auch nach längerer Prozeßdauer konnte keine Überalterung und somit geringere Aktivität der Biomasse festgestellt werden. Nennenswerte Infektionen sind auch nach mehrwöchiger Betriebszeit nicht nachweisbar. Diese Umstände sind mit gleichbleibend guten Bedingungen für den Mikroorganismus zu erklären. Sowohl die Konzentration der zucker- oder -stärkehaltigen Substanz im Fermenter, welche niedrig gehalten wurde, als auch der osmotische Druck des Fermentationsfluides, der mit ca. 30 bar sehr klein war, wirken nicht hemmend auf Zellwachstum und Produktbildung.

Osmotisch wirksame Substanzen und toxische Nebenprodukte wurden kontinuierlich mit dem Permeat-Stoffstrom aus der Fermentation entfernt.

## Ansprüche

1. Verfahren zur fermentativen Erzeugung organischer Lösungsmittel wie Butanol, Azeton, insbesondere von Ethanol, durch kontinuierliche Fermentation eines zucker- oder stärkehaltigen wässrigen Mediums, welches neben den fermentierbaren auch lösliche und nichtlösliche nichtfermentierbare Substanzen enthalten kann, wobei die organischen Lösungsmittel, insbesondere Ethanol, sowohl als Stoffwechselprodukte von Hefen als auch von Bakterien gebildet werden, d a - d u r c h  g e k e n n z e i c h n e t , daß

a) ihre Entfernung aus dem Prozeß mittels zweier Stoffablaufströme unterschiedlicher Zusammensetzung erfolgt,

b) einer der Stoffströme das Permeat darstellt, welches durch Membranfiltration gewonnen wird und weitestgehend frei ist von vegetativen Hefe- und/oder Bakterienzellen, Feststoffpartikeln und Substanzen mit molarer Masse oberhalb 5.000,

c) der andere Stoffstrom als Fermenter-Stoffstrom direkt aus dem Fermentationsfluid entnommen wird und den größten Teil der durch das zucker- oder stärkehaltige wässrige Medium zugeführten nichtlöslichen nichtfermentierbaren Substanzen sowie die produktbildende Biomasse in hoher Konzentration enthält und gegenüber dem Permeat-Stoffstrom kleiner gehalten wird.

2. Verfahren nach Anspruch 1, d a d u r c h  g e - k e n n z e i c h n e t , daß der Permeat-Stoffstrom ein Vielfaches des Fermenter-Stoffstromes beträgt.

3. Verfahren nach Anspruch 1 oder 2, d a d u r c h  g e k e n n z e i c h n e t , daß durch Wahl der Größe des Fermenter-Stoffstromes eine entsprechend hohe Konzentration der produktbildenden Biomasse im Fermentationsfluid eingestellt wird, die mittels natürlicher Selektion Infektionen durch andere Mikroorganismen verhindert.

4. Verfahren nach einem der Ansprüche 1 bis 3, d a - d u r c h  g e k e n n z e i c h n e t , daß die Konzentration der produktbildenden Biomasse so eingestellt wird, daß bei hohen Durchsatzraten Produktkonzentrationen erreicht werden, die den in batch-Fermentationen erzielbaren entsprechen.

5. Verfahren nach einem der Ansprüche 1 bis 4, g e - k e n n z e i c h n e t  d u r c h  Verwendung eines Membranfilters mit physikalischen Eigenschaften, die ein Ausschleusen von osmotisch wirksamen Substanzen und toxischen Nebenprodukten, die die Fermentation hemmen, als Bestandteile des Permeat-Stoffstromes ermöglichen.

6. Verfahren nach einem der Ansprüche 1 bis 5, d a - d u r c h  g e k e n n z e i c h n e t , daß unabhängig von der Zusammensetzung des zucker- oder stärkehaltigen wässrigen Mediums der Fermenter-Stoffstrom so eingestellt wird, daß die Gesamttrockensubstanzgehalte des Fermentationsfluides wenigstens 18 Gew.% erreichen.

7. Verfahren nach einem der Ansprüche 1 bis 7, d a - d u r c h  g e k e n n z e i c h n e t , daß die Abtrennung der organischen Lösungsmittel aus den beiden unterschiedlichen Stoffströmen getrennt vorgenommen wird.

Europäisches
Patentamt

**EUROPÄISCHER RECHERCHENBERICHT**

Nummer der Anmeldung

0195094

EP 85 10 3044

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | WO-A-8 501 064 (M. CHERYAN u.a.) <br> * Figur 1; Anspruch 10; Seite 18, letzter Absatz; Seite 19 * <br> --- | 1,5,7 | C 12 M .1/12 <br> C 12 P 7/06 <br> C 12 P 7/16 <br> C 12 P 7/28 |
| A | BIOTECHNOLOGY AND BIOENGINEERING, Band XX, 1978, Seiten 709-753, John Wiley & Sons Inc., New York, US; A. MARGARITIS u.a.: "The rotorfermentor. I. Description of the apparatus, power requirements, and mass transfer characteristics. II. Application to ethanol fermentation" <br> * Seiten 710-712,720,727,734-737 * <br> ----- | 1,5 | |

RECHERCHIERTE SACHGEBIETE (Int. Cl.4)

C 12 M

Der vorliegende Recherchenbericht wurde für alle Patentansprüche erstellt.

| Recherchenort | Abschlußdatum der Recherche | Prüfer |
|---|---|---|
| DEN HAAG | 06-12-1985 | NESTBY K. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, überein- stimmendes Dokument

EPA Form 1503. 03 82